# FASCICULE DE BREVET EUROPEEN

(11) **EP 1 553 943 B1**
(45) Date de publication et mention de la délivrance du brevet: **14.05.2008**
(21) Numéro de dépôt: 03778427.9
(22) Date de dépôt: 21.10.2003
(51) Int. Cl.: A61K 31/444, A61P 1/04, A61P 11/00, A61P 11/04, A61P 11/06, A61P 11/14

(54) **UTILISATION DU TENATOPRAZOLE POUR LE TRAITEMENT DU REFLUX GASTRO-OESOPHAGIEN**
VERWENDUNG VON TENATOPRAZOL ZUR BEHANDLUNG VON GASTROÖSOPHOGALEM REFLUX
USE OF TENATOPRAZOLE FOR THE TREATMENT OF GASTROESOPHAGEAL REFLUX

(30) Priorité: 21.10.2002 FR 0213113
(43) Date de publication de la demande: 20.07.2005
(60) Demande divisionnaire: 08000893.1
(73) Titulaire: Sidem Pharma SA, 2613 Luxembourg (LU); Mitsubishi Pharma Corporation, Osaka-shi, Osaka 541-0046 (JP)
(72) Inventeur: SCHUTZE, François, F-78860 SAINT-NOM-LA-BRETECHE (FR); CHARBIT, Suzy, F-94000 CRETEIL (FR); FICHEUX, Hervé, F-94130 NOGENT-SUR-MARNE (FR); HOMERIN, Michel, F-91080 COURCOURONNES (FR); TACCOEN, Alain, F-78150 LE CHESNAY (FR); INABA, Yoshio, TOKYO, Tokyo 103-8405 (JP)
(74) Mandataire: L'Helgoualch, Jean
(86) Numéro de dépôt international: PCT/FR2003/003122
(87) Numéro de publication internationale: WO 2004/037255

(56) Documents cités:
- WO-A-00/50037
- WO-A-01/28558
- WO-A-98/16228
- WO-A-99/59544
- WO-A-02/072070
- ANON.: "Tenatoprazole: benatoprazole, TU 199" DRUGS IN R&D (2002), 3(4), 276-277, XP008018955
- UCHIYAMA KAZUYUKI ET AL: "The long-lasting effect of TU-199, a novel H+,K+-ATPase inhibitor, on gastric acid secretion in dogs." JOURNAL OF PHARMACY AND PHARMACOLOGY, vol. 51, no. 4, avril 1999 (1999-04), pages 457-464, XP008018962 ISSN: 0022-3573
- HOWDEN C W ET AL: "Appropriate acid suppression for optimal healing of duodenal ulcer and gastro-oesophageal reflux disease." SCANDINAVIAN JOURNAL OF GASTROENTEROLOGY, vol. 29, no. SUPPL. 201, 1994, pages 79-82, XP008018957 ISSN: 0036-5521 cité dans la demande
- WILKINSON S P ET AL: "Regression of columnar-lined (Barrett's) oesophagus with omeprazole 40 mg daily: Results of 5 years of continuous therapy." ALIMENTARY PHARMACOLOGY & THERAPEUTICS, vol. 13, no. 9, 1999, pages 1205-1209, XP002246271 ISSN: 0269-2813
- HENDEL J ET AL: "Morning or evening dosage of omeprazole for gastro-oesophageal reflux disease?" ALIMENTARY PHARMACOLOGY & THERAPEUTICS, vol. 9, no. 6, 1995, pages 693-697, XP008018964 ISSN: 0269-2813
- DE CAESTECKER JOHN: "Medical therapy for supraesophageal complications of gastroesophageal reflux." AMERICAN JOURNAL OF MEDICINE, vol. 103, no. 5 PART A, 24 novembre 1997 (1997-11-24), pages 138S-143S, XP001159192 ISSN: 0002-9343
- CENGIA GIANPAOLO ET AL: "Nocturnal chronic cough and GERD: Clinical correlations and significance of abnormal proximal esophageal reflux." GASTROENTEROLOGY, vol. 116, no. 4 PART 2, avril 1999 (1999-04), page A134 XP008018958 Digestive Disease Week and the 100th Annual Meeting of the American Gastroenterological Association;Orlando, Florida, USA; May 16-19, 1999 ISSN: 0016-5085
- MAY B ET AL: "Extraesophageal manifestations of reflux disease concerning the airways." VERDAUUNGSKRANKHEITEN, vol. 16, no. 6, novembre 1998 (1998-11), pages 267-270, XP008018331 ISSN: 0174-738X
- WORTH H ET AL: "Interrelationships between gastro-esophageal reflux and airway diseases-pathophysiology, diagnostic and therapeutic possibilities." ATEMWEGS- UND LUNGENKRANKHEITEN, vol. 20, no. 12, 1994, pages 697-700, XP008018329 ISSN: 0341-3055
- RODRIGUEZ-TELLEZ M ET AL: "Posterior laryngitis: Effects of treatment with omeprazole alone." REVISTA ESPANOLA DE ENFERMEDADES DIGESTIVAS, vol. 94, no. 3, 2002, pages 127-130, XP008018960 ISSN: 1130-0108
- FITZGERALD R.C. ET AL: 'Review Article: Barrett's oesophagus, dysplasia and pharmacologic acid suppression' ALIMENT. PHARMACOL. THER. vol. 15, 2001, pages 269 - 276
- FRAZZONI ET AL: 'Different patterns of oesophageal acid exposure distinguish complicated reflux disease from either erosive reflux oesophagitis or non-erosive reflux disease' ALIMENT. PHARMACOL. THER. vol. 18, Février 2003, pages 1091 - 1098
- SACHS G. ET AL: 'Review article: the clinical pharmacology of proton pump inhibitors' ALIMENT. PHARMACOL. THER. vol. 23, 2006, pages 2 - 8
- SCARPIGNATO C. ET AL: 'Acid suppression therapy: where do we go from here?' DIGESTIVE DISEASES vol. 24, 2006, pages 11 - 46

## Description

La présente invention concerne le traitement de maladies liées au reflux gastro-oesophagien, et plus particulièrement l'utilisation du ténatoprazole dans la fabrication d'un médicament destiné au traitement du reflux gastro-oesophagien nocturne et de l'oesophage de Barrett.

Le ténatoprazole, c'est-à-dire la 5-méthoxy-2-[[(4-méthoxy-3,5-diméthyl-2-pyridyl)méthyl]sulfinyl]imidazo[4,5-b]pyridine, est décrit dans le brevet EP 254.588. Il fait partie des médicaments considérés comme des inhibiteurs de la pompe à protons, utiles pour le traitement des ulcères gastriques et duodénaux. Parmi les autres inhibiteurs de la pompe à protons, on peut citer l'oméprazole, le rabéprazole, le pantoprazole, le lansoprazole, qui présentent tous une analogie structurelle, et se rattachent au groupe des pyridinyl-méthyl-sulfinyl-benzimidazoles. Ces composés sont des sulfoxydes présentant une asymétrie au niveau de l'atome de soufre et sont donc généralement sous forme de mélange racémique de deux énantiomères.

Le premier dérivé connu de cette série est l'oméprazole, décrit dans le brevet EP 005.129, qui possède des propriétés inhibitrices de la sécrétion acide gastrique, et est largement utilisé comme anti-ulcéreux en thérapeutique humaine.

L'oméprazole a aussi été envisagé dans le traitement des troubles du reflux gastro-oesophagien, mais son action dans une telle indication n'est pas totalement satisfaisante. Ainsi, des études ont montré que sa durée d'action, comme dans le cas des autres inhibiteurs de la pompe à protons, est insuffisante pour traiter efficacement le reflux nocturne.

Le reflux gastro-oesophagien est considéré comme lié essentiellement à un désordre de la motilité caractérisé par un relâchement transitoire anormalement fréquent et une perte de tonus du sphincter du bas oesophage. Ces anomalies ont pour effet de permettre un reflux du contenu de l'estomac dans l'oesophage. En outre, chez les patients souffrant de reflux gastro-oesophagien, l'élimination de l'acidité du reflux est en moyenne 50% plus lente que chez un sujet normal et la résistance de la paroi oesophagienne à l'agression acide est sensiblement diminuée. Aussi, la sécrétion acide de l'estomac joue un rôle majeur dans l'apparition et la persistance des lésions de la muqueuse oesophagienne des patients souffrant de reflux gastro-oesophagien.

Diverses études ont montré que la sévérité des symptômes chez les patients souffrant de reflux gastro-oesophagien est proportionnelle à la durée d'exposition de la muqueuse oesophagienne à l'acide (Howden CW, Burget DW, Hunt RH "Appropriate acid suppression for optimal healing of duodenal ulcer and gastro-oesophageal reflux disease", Scand. J. Gastroenterol, Suppl (1994) 201:79-82). Ainsi, les sujets non symptomatiques ont une exposition d'environ 1% (pourcentage de temps d'exposition à l'acidité en une journée), tandis que ceux qui sont affectés occasionnellement de reflux gastro-oesophagien ont un taux d'exposition voisin de 2%, les sujets avec symptômes quotidiens un taux de 3% et les patients présentant des lésions endoscopiques un taux variant de 6% à 12% selon la gravité de la lésion. Ces études ont été faites pour des expositions à une acidité de pH inférieur à 4, c'est-à-dire anormalement bas au niveau de l'oesophage où les valeurs normales sont généralement comprises entre 5 et 7.

Ces études ont donc montré que plus l'exposition acide est longue, plus les symptômes et les lésions de la muqueuse oesophagienne sont sévères.

De plus, les études ont montré que la suppression de l'acide résultant d'un traitement médical approprié est corrélée avec le taux de guérison des lésions, les paramètres importants étant la durée de l'inhibition acide et son amplitude. C'est pourquoi on a souvent prescrit aux patients souffrant de reflux gastro-oesophagien, l'administration de médicaments antiacides ou d'antagonistes des récepteurs à l'histamine ou encore d'inhibiteurs de la pompe à protons en vue d'obtenir un soulagement des symptômes. Cependant, la plupart des médicaments utilisés ne sont pas pleinement satisfaisants car ils ne procurent qu'un soulagement partiel des symptômes, ou ils ont une durée d'action trop courte, impliquant des prises répétées de médicament.

Diverses techniques ont été mises au point pour obtenir des formulations contenant des inhibiteurs de la pompe à protons susceptibles de procurer des propriétés améliorées. Par exemple, WO 02.072070 décrit des microparticules obtenues par une technique de lyophilisation par pulvérisation, possédant une teneur élevée en sel de magnésium d'oméprazole ou d'esoméprazole, qui peuvent être enduites par une couche gastro-résistante pour les protéger du contact avec le jus gastrique acide. Un autre composé préféré est le ténatoprazole. WO 99.59544 décrit des comprimés désintégrable pour administration par voie orale contenant de fins granulés comprenant une composition enduite par une couche gastro-résistante. Cette composition comprend une substance active telle que le lansoprazole. Les microparticules et comprimés contenant de fins granulés décrits dans ces documents sont supposés être utiles dans les traitement usuels des affections liées à l'acidité gastrique, mais aucun résultat clinique n'est fourni. Adis R&D Profile (2002:3(4) 276-277) relate certaines propriétés du ténatoprazole et signale qu'il est enregistré au Japon pour le reflux oesophagien en Avril 2002, mais cette information est erronée et l'enregistrement n'a jamais été délivré car la démonstration de cette indication possible du ténatoprazole n'a jamais été faite à l'époque.

R. C. Fitzgerald et al. suggèrent dans Aliment. Pharmacol. Ther., 15, 269-276 (2001) que la capacité des inhibiteurs de pompes à protons à supprimer l'acide oesophagien de façon profonde et uniforme pourrait être critique dans le traitement de longue durée de l'oesophage de Barrett.

Il subsiste donc aujourd'hui un besoin d'un médicament susceptible de traiter et soulager efficacement les patients souffrant de reflux gastro-oesophagienlen nocturne et de l'oesophage de Barrett.

Les études et expérimentations effectuées par la demanderesse ont montré de manière inattendue que le ténatoprazole pouvait être utilisé efficacement dans le traitement des maladies liées au reflux gastro-oesophagien notamment du reflux gastro-oesophagien nocturne et de l'oesophage de Barrett, alors que l'oméprazole et les autres inhibiteurs de la pompe à protons de structure analogue ne procurent pas une efficacité de traitement satisfaisante dans ces indications.

La présente invention a donc pour objet l'utilisation du ténatoprazole dans la fabrication d'un médicament pour le traitement du reflux gastro-oesophagien nocturne et l'oesophage de Barrett.

Comme l'oméprazole et les autres sulfoxydes à structure analogue, le ténatoprazole comporte une structure asymétrique et peut donc se présenter sous la forme du mélange racémique ou de ses énantiomères.

Contrairement à tous les autres inhibiteurs de la pompe à protons tels que, par exemple, l'oméprazole ou l'esoméprazole, et de manière inattendue, le ténatoprazole possède une durée d'action nettement prolongée, résultant d'une demi-vie environ 7 fois supérieure. Ainsi, les données médicales recueillies montrent que le ténatoprazole apporte un niveau de soulagement des symptômes et de cicatrisation des lésions supérieur à celui des autres médicaments appartenant à la même classe thérapeutique des inhibiteurs de la pompe à protons.

La présente invention permet d'utiliser le ténatoprazole pour apporter un niveau supérieur de soulagement des symptômes atypiques nocturnes qui sont encore aujourd'hui souvent réfractaires aux traitements par les inhibiteurs de la pompe à protons usuels, tels que l'oméprazole.

Un autre avantage de la présente invention est que le ténatoprazole peut aussi agir efficacement sur la maladie de l'oesophage de Barrett, ou endobrachyoesophage, qui est définie par la présence d'une muqueuse de type intestinal (cylindrique) au niveau du bas oesophage ou de la jonction gastro-oesophagienne. Cette affection est une complication de l'oesophagite peptique, qui peut résulter du reflux gastro-oesophagien, et elle peut dégénérer dans certains cas en adénocarcinome.

Les malades souffrant de l'oesophage de Barrett ont en général un reflux gastro-oesophagien plus important que la moyenne, et l'importance de l'acidité du reflux peut avoir des effets néfastes sur la différentiation et la prolifération cellulaire, pouvant favoriser le développement d'une dysplasie. Il est donc important de pouvoir réduire la sécrétion acide chez les patients présentant des symptômes liés au reflux gastro-oesophagien avec des lésions histologiques en rapport avec un oesophage de Barrett.

Le traitement doit procurer une suppression maximale de l'acidité du reflux gastro-oesophagien, dans le cas de l'oesophage de Barrett, et l'administration de ténatoprazole permet précisément d'y parvenir, et plus particulièrement de prévenir les poussées acides nocturnes, ce que les médicaments actuellement disponibles ne permettent pas d'obtenir, même les inhibiteurs de la pompe à protons classiques.

Comme indiqué ci-dessus, le ténatoprazole se distingue des autres inhibiteurs de la pompe à protons par une demi-vie d'élimination étonnamment plus longue, et aussi par une exposition tissulaire importante, comme l'ont démontré les expérimentations effectuées par la demanderesse.

L'étude de phase I chez des individus de type causasien (n=8 par groupe) a permis de montrer l'influence de différentes doses de ténatoprazole sur les paramètres pharmacocinétiques, dans le cas d'une administration par voie orale en une dose unique, et pendant une période de 7 jours.

Les doses testées sont de 10, 20, 40 et 80 mg de ténatoprazole.

Les résultats obtenus sont regroupés au Tableau 1 ci-après.

**Tableau 1**

| | Dose unique | | | | Dose répétée (7 jours) | | | |
|---|---|---|---|---|---|---|---|---|
| | 10 mg | 20 mg | 40 mg | 80 mg | 10 mg | 20 mg | 40 mg | 80 mg |
| Cmax (µg/ml) | 0,9 | 2,4 | 5,3 | 8,3 | 1,6 | 3 | 5,5 | 11,8 |
| Tmax (h) | 4 | 4 | 3 | 3 | 3 | 2 | 3 | 2 |
| T1/2 (h) | 5 | 6 | 6 | 7 | 5 | 8 | 9 | 9,2 |
| AUC 0-t | 8 | 24 | 43 | 97 | 13 | 36 | 75 | 218 |

Dans ce tableau, les abréviations utilisées ont les signification suivantes :
- Cmax: concentration maximale
- Tmax: temps pour obtenir la concentration maximale
- T1/2: temps de demi-vie d'élimination
- AUC₀₋ₜ: aire sous la courbe, entre le temps 0 et la dernière concentration mesurable.

Les résultats exposés au Tableau 1 ci-dessus montrent que les moyennes de temps de demi-vie d'élimination sont comprises entre 5 et 6 heures après administration d'une dose unique, et entre 5 et 9,5 heures après 7 jours d'administration, selon la dose. Le ténatoprazole présente aussi de fortes valeurs d'AUC (aire sous la courbe) mettant en évidence un faible taux de métabolisme et/ou une forte biodisponibilité par voie orale. De plus, quelles que soient les conditions d'administration, unique ou répétée, les valeurs de Cmax, AUC₀₋ₜ et AUC_{0-inf} augmentent de manière linéaire. La valeur de AUC_{0-inf} est calculée par extrapolation.

Une comparaison des valeurs d'AUC entre deux inhibiteurs de la pompe à protons, le lansoprazole et l'oméprazole, a déjà été faite par Tolman et al. (J. Clin. Gastroenterol., 24(2), 65-70, 1997) mais elle ne permet pas de juger de la supériorité d'un produit par rapport à un autre. En effet, différents critères entrent en jeu, à savoir le temps de régénération de la pompe, et le temps passé au-dessus de la concentration minimale nécessaire pour inhiber les pompes à protons. En ce qui concerne le temps de régénération des pompes, on observe que les pompes ont généralement une durée de demi-vie de l'ordre de 30 à 48 heures, et elles sont donc renouvelées totalement toutes les 72 à 96 heures.

Grâce aux propriétés pharmacocinétiques exposées ci-dessus, le ténatoprazole permet de s'opposer au phénomène de régénération des pompes à protons en maintenant une concentration inhibitrice sur une période de temps suffisamment longue pour répondre aux deux critères précités.

Ainsi, l'exposition prolongée liée a la longue demi-vie d'élimination du ténatoprazole, mise en évidence par la valeur d'AUC, lui confère une plus longue présence au niveau des sites d'action et procure donc un effet pharmacodynamique prolongé dans le temps. Les expérimentations montrent ainsi que le ténatoprazole possède un rapport demi-vie plasmatique / temps de régénération des pompes notablement plus élevé que celui des autres inhibiteurs de la pompe à protons, ce qui permet de l'utiliser dans des pathologies où les médicaments actuels sont peu efficaces, en particulier dans le traitement de l'oesophage de Barrett.

Le ténatoprazole peut être administré sous les formes usuelles adaptées au mode d'administration choisi, par exemple par voie orale ou parentérale, de préférence par voie orale ou intraveineuse. On peut utiliser par exemple des formulations de comprimés ou de gélules contenant le ténatoprazole comme principe actif, ou encore des solutés buvables ou des émulsions ou solutions pour administration parentérale contenant un sel de ténatoprazole avec un support pharmaceutiquement acceptable usuel. Le sel de ténatoprazole peut être choisi parmi les sels de sodium, de potassium, de magnésium ou de calcium.

A titre d'exemple, une formulation appropriée de comprimés contenant 20 mg de ténatoprazole associé à des supports et excipients pharmaceutiquement acceptables, est indiquée ci-dessous :

| | | |
|---|---|---|
| Ténatoprazole | | 20,0 mg |
| lactose | | 32,0 mg |
| hydroxyde d'aluminium | | 17,5 mg |
| hydroxypropylcellulose | | 12,1 mg |
| talc | | 4,5 mg |
| dioxyde de titane | | 3,2 mg |
| stéarate de magnésium | | 1,0 mg |
| excipients usuels | q.s.p. | 160 mg |

La posologie est déterminée par le praticien en fonction de l'état du patient et de la gravité de l'affection. Elle est généralement comprise entre 10 et 120 mg, de préférence entre 20 et 40 mg, de ténatoprazole par jour, correspondant par exemple à une prise de 1 à 2 comprimés contenant chacun 20 ou 40 mg de principe actif par jour pendant une période de temps qui peut être comprise entre 4 et 12 semaines, dans le cas d'un traitement d'attaque ou d'entretien. Dans le cas d'une forme pédiatrique adaptée à des enfants en bas âge, par exemple sous forme de soluté buvable, la dose unitaire peut être plus faible, par exemple 2 ou 5 mg. Dans le cas d'affections sévères, il peut être efficace d'administrer le médicament dans un premier temps par voie intraveineuse, puis par voie orale. L'invention présente en outre l'avantage de permettre un traitement séquentiel efficace par simple administration d'un seul comprimé dosé à 20 ou 40 mg par semaine.

Des exemples cliniques sont donnés ci-après, montrant les effets du traitement sur des patients souffrant de reflux gastro-oesophagien ou de dyspepsie (référence), traités par administration de ténatoprazole par voie orale.

**Tableau 2**

| Traitement de patients avec symptômes de reflux | | | | |
|---|---|---|---|---|
| gastro-oesophagien | | | | |
| Age/Sexe | Symptôme | Durée de | Evolution du | Tolérance |
| | prédominant | traitement | symptôme | |
| 47/F | b.n. | 8 semaines | ++ | +++ |
| 38/M | b. | 8 semaines | +++ | +++ |
| 35/F | b.n. | 4 semaines | ++ | +++ |
| 34/M | b. | 8 semaines | +++ | +++ |
| 45/M | b.n. | 8 semaines | +++ | +++ |
| 30/M | b.n. | 8 semaines | +++ | ++ |
| 49/F | r. | 8 semaines | ++ | +++ |
| 42/M | b. | 8 semaines | ++ | +++ |
| 38/F | b.n. | 8 semaines | +++ | +++ |
| 25/F | b. | 12 semaines | +++ | +++ |
| 28/M | b.n. | 4 semaines | +++ | +++ |
| 39/F | b.n. | 4 semaines | + | +++ |
| 41/M | b. | 8 semaines | +++ | +++ |
| 36/F | r. | 8 semaines | +++ | ++ |

| | | | | |
|---|---|---|---|---|
| b. : brûlures b.n.: brûlures nocturnes r. régurgitations | | | | |

Les symboles + à +++ identifient une évolution du symptôme et une tolérance de valeur moyenne à très favorable.

Le traitement consiste en une administration quotidienne d'un comprimé dosé à 20 mg de ténatoprazole. Le tableau montre que le traitement est parfaitement toléré dans 12 cas sur 14 et bien toléré dans les deux autres, tandis que l'évolution constatée des symptômes a été généralement très favorable.

**Tableau 3**

| Traitement de patients avec symptômes atypiques | | | | | |
|---|---|---|---|---|---|
| de reflux gastro-oesophagien (référence) | | | | | |
| Age/Sexe | Symptôme | liaison | Durée de | Evolution du | Tolérance |
| | prédominant | avec gerd | traitement | symptôme | |
| 44/M | pharyngite | + | 4 semaines | ++ | +++ |
| 36/M | dysphonie | ++ | 5 semaines | +++ | +++ |
| 34/F | dysphonie | ++ | 4 semaines | ++ | +++ |
| 45/M | pseudo angor | ++ | 8 semaines | +++ | +++ |
| 29/F | toux nocturne | +++ | 7 semaines | +++ | +++ |
| 27/M | carie dentaire | + | 12 semaines | 0 | ++ |
| 33/M | asthme | ++ | 12 semaines | ++ | +++ |
| 34/F | pharyngite | ++ | 8 semaines | ++ | +++ |
| 36/F | toux nocturne | ++ | 8 semaines | +++ | ++ |
| 26/M | asthme | ++ | 12 semaines | +++ | +++ |
| 49/M | pseudo angor | ++ | 12 semaines | +++ | +++ |
| 31/F | pharyngite | + | 8 semaines | + | +++ |

| | | | | | |
|---|---|---|---|---|---|
| gerd : troubles du reflux gastro-oesophagien. | | | | | |

Les résultats du tableau ci-dessus montrent que l'évolution des symptômes est particulièrement favorable dans les cas où le lien avec le reflux gastro-oesophagien est plus net.

**Tableau 4**

| Traitement de patients avec symptômes de | | | | |
|---|---|---|---|---|
| dyspepsie fonctionnelle (référence) | | | | |
| Age/Sexe | Symptôme | Durée de | Evolution du | Tolérance |
| | prédominant | traitement | symptôme | |
| 47/F | n. | 4 semaines | ++ | +++ |
| 38/M | p.g. | 8 semaines | +++ | +++ |
| 35/F | b. | 8 semaines | +++ | +++ |
| 34/F | s.p. | 8 semaines | +++ | +++ |
| 45/M | d.e. | 6 semaines | +++ | ++ |
| 30/F | b.n. | 8 semaines | +++ | +++ |
| 49/F | n. | 8 semaines | ++ | +++ |
| 42/M | s.p. | 6 semaines | ++ | +++ |
| 38/F | d.e. | 8 semaines | +++ | ++ |
| 25/F | g.e. | 12 semaines | ++ | +++ |
| 28/M | s.b. | 4 semaines | + | +++ |
| 39/F | d.e. | 4 semaines | ++ | ++ |
| 41/M | b. | 6 semaines | +++ | +++ |
| 36/F | g.e. | 8 semaines | +++ | ++ |
| 44/F | n. | 10 semaines | +++ | +++ |

| | | | | |
|---|---|---|---|---|
| b. : brûlures b.n.: brûlures nocturnes n. : nausées p.g.: sensations de plénitude gastrique s.p.: satiété précoce d.e.: douleur épigastrique g.e.: gêne épigastrique s.b.: sensation de ballonnement | | | | |

Ces résultats confirment l'efficacité du ténatoprazole, dans le traitement de la dyspepsie.

Deux études ouvertes ont été réalisées pour évaluer l'efficacité et la sécurité du ténatoprazole dans le traitement du reflux gastro-oesophagien. A 22 patients dans la première étude et 24 patients dans la seconde, âgés de plus de 20 ans, souffrant de reflux oesophagien érosif et/ou ulcéré (diagnostiqué par endoscopie), on a administré des comprimés de granulés à enrobage gastro-résistant contenant 10 mg de ténatoprazole.

Les comprimés ont été administrés par voie orale, une fois par jour après le petit déjeuner, pendant une période de traitement de 8 semaines, qui a été prolongée jusqu'à 12 semaines dans certains cas. La guérison a été suivie par examen endoscopique, 4 semaines et 8 semaines après la première administration, ou au retrait du cas de l'étude, les degrés de la maladie ont été évalués suivant la classification de Savary et Miller, et le traitement a été arrêté quand la guérison était confirmée. L'état a été évalué comme guéri quand la disparition de l'érosion était confirmée.

Le degré d'amélioration endoscopique a été évalué suivant les 6 niveaux suivants du degré de l'affection : "guéri", "fortement diminué", modérément diminué", "légèrement diminué", "inchangé" et "aggravé".

Le degré d'amélioration des symptômes subjectifs et objectifs, comparés avec ceux observés au départ de l'étude, a été évalué suivant les 6 niveaux suivants : "fortement amélioré", "modérément amélioré", "légèrement amélioré", "inchangé", "aggravé" et "aucun symptôme depuis le début de l'étude".

La guérison a été observée à 4 semaines et l'administration du ténatoprazole a été arrêtée à ce moment dans 20 cas dans la première étude et 23 cas dan la seconde. Un seul patient n'a pas été guéri au bout de 8 semaines de traitement.

Les résultats sont indiqués aux Tableaux 5 et 6 ci-après.

**Tableau 5**

| **Amélioration du Grade Endoscopique** | | | | | | | |
|---|---|---|---|---|---|---|---|
| | A | B | C | D | E | F | Total |
| 1^{ère} étude | | | | | | | |
| 4 sem. | 20 | 1 | 0 | 0 | 1 | 0 | 22 |
| 8 sem. | 21 | 0 | 0 | 0 | 1 | 0 | 22 |
| terme | 21 | 0 | 0 | 0 | 1 | 0 | 22 |

| 2^{ème} étude | | | | | | | |
|---|---|---|---|---|---|---|---|
| 4 sem. | 23 | 2 | 0 | 0 | 0 | 0 | 25 |
| 8 sem. | 24 | 0 | 0 | 0 | 0 | 0 | 24 |
| 12 sem. | 24 | 0 | 0 | 0 | 0 | 0 | 24 |
| terme | 24 | 0 | 0 | 0 | 0 | 0 | 24 |

| | | | | | | | |
|---|---|---|---|---|---|---|---|
| A : guéri B : fortement diminué C : modérément diminué D : légèrement diminué E : inchangé F : aggravé | | | | | | | |

**Tableau 6**

| **Amélioration du niveau d'intensité des symptômes** | | | | | | |
|---|---|---|---|---|---|---|
| | A | B | C | D | E | Total |
| 1^{ère} étude | | | | | | |
| 1 sem. | 16 | 6 | 0 | 3 | 0 | 25 |
| 2 sem. | 21 | 1 | 0 | 1 | 0 | 23 |
| 4 sem. | 23 | 0 | 1 | 0 | 0 | 24 |
| 6 sem. | 10 | 0 | 1 | 0 | 0 | 11 |
| 8 sem. | 9 | 0 | 1 | 0 | 0 | 10 |
| 10 sem. | 2 | 0 | 0 | 0 | 0 | 2 |
| Final | 21 | 0 | 1 | 0 | 0 | 22 |

| 2^{ème} study | | | | | | |
|---|---|---|---|---|---|---|
| 1 sem. | 19 | 2 | 1 | 1 | 0 | 23 |
| 2 sem. | 21 | 1 | 1 | 0 | 0 | 23 |
| 4 sem. | 20 | 1 | 1 | 0 | 0 | 22 |
| 6 sem. | 10 | 0 | 1 | 0 | 0 | 11 |
| 8 sem. | 10 | 1 | 0 | 0 | 0 | 11 |
| 10 sem. | 0 | 1 | 0 | 0 | 0 | 1 |
| 12 sem. | 0 | 1 | 0 | 0 | 0 | 1 |
| Final | 19 | 2 | 0 | 0 | 0 | 21 |

| | | | | | | |
|---|---|---|---|---|---|---|
| A : fortement amélioré B : modérément amélioré C : légèrement amélioré D : inchangé E : aggravé | | | | | | |

Les résultats ci-dessus démontrent que le ténatoprazole suivant la présente invention est très efficace dans le traitement du reflux gastro-oesophagien, car la guérison est obtenue par un traitement de 4 semaines, à comparer aux résultats insatisfaisants obtenus dans des traitements de 8 semaines avec les inhibiteurs de la pompe à protons usuels.

## Revendications

1. Utilisation du ténatoprazole dans la fabrication d'un médicament pour le traitement du reflux gastro-oesophagien nocturne et de l'oesophage de Barrett.

2. Utilisation selon la revendication 1, **caractérisée en ce que** le ténatoprazole est pour administration par voie orale.

3. Utilisation selon la revendication 1, **caractérisée en ce que** le ténatoprazole est pour administration par voie parentérale.

4. Utilisation selon l'une quelconque des revendications précédentes, **caractérisée en ce que** le ténatoprazole est pour administration à raison de 10 à 120 mg par jour.

5. Utilisation selon la revendication 4, **caractérisée en ce que** le ténatoprazole est présenté en dose unitaire contenant 20 à 40 mg de principe actif, associé à un ou plusieurs excipients et supports pharmaceutiquement acceptables.

6. Utilisation selon l'une quelconque des revendications précédentes, **caractérisée en ce que** le ténatoprazole est sous forme de sel de sodium, de potassium, de magnésium ou de calcium.

## Claims

1. The use of tenatoprazole in the manufacture of a medicament for the treatment of nocturnal gastroesophageal reflux and Barrett's oesophagus.

2. Use according to claim 1, **characterized in that** tenatoprazole is for administration via the oral route.

3. Use according to claim 1, **characterized in that** tenatoprazole is for administration via the parenteral route.

4. Use according to any of the preceding claims, **characterized in that** tenatoprazole is for administration at a rate of 10 to 120 mg per day.

5. Use according to claim 4, **characterized in that** tenatoprazole is presented in a unit dosage form containing 20 to 40 mg of active substance, associated with one or several pharmaceutically acceptable excipients and substrates.

6. Use according to any preceding claims, **characterized in that** the medicament is presented as a salt of sodium, potassium, magnesium or calcium.

## Patentansprüche

1. Verwendung von Tenatoprazol zur Herstellung eines Medikaments zur Behandlung von nächtlichem gastroösophagealem Reflux und Barrett-Ösophagus.

2. Verwendung nach Anspruch 1, **dadurch gekennzeichnet, dass** das Tenatoprazol zur oralen Verabreichung dient.

3. Verwendung nach Anspruch 1, **dadurch gekennzeichnet, dass** das Tenatoprazol zur parenteralen Verabreichung dient.

4. Verwendung nach einem der vorangegangenen Ansprüche, **dadurch gekennzeichnet, dass** das Tenatoprazol in einer Menge von 10 bis 120 mg pro Tag verabreicht wird.

5. Verwendung nach Anspruch 4, **dadurch gekennzeichnet, dass** das Tenatoprazol in einer Einheitsdosis vorliegt, die 20 bis 40 mg des Wirkstoffs enthält, und mit einem oder mehreren Exzipienten und pharmazeutisch annehmbaren Trägern assoziiert ist.

6. Verwendung nach einem der vorangegangenen Ansprüche, **dadurch gekennzeichnet, dass** das Tenatoprazol in Form des Natrium-, Kalium-, Magnesium- oder Calciumsalzes vorliegt.
